# EUROPEAN PATENT SPECIFICATION

(11) **EP 4 034 238 B1**
(45) Date of publication and mention of the grant of the patent: **24.09.2025**
(21) Application number: 20786476.0
(22) Date of filing: 23.09.2020
(51) Int. Cl.: A61P 9/10, C07K 16/40

(54) **ANTIBODIES FOR THE DIAGNOSIS AND/OR TREATMENT OF ATHEROSCLEROSIS**
VERFAHREN ZUR DIAGNOSE UND/ODER BEHANDLUNG VON ARTERIOSKLEROSE
ANTICORPS POUR DIAGNOSTIC ET/OU TRAITEMENT DE L'ATHÉROSCLÉROSE

(30) Priority: 23.09.2019 EP 19382816
(43) Date of publication of application: 03.08.2022
(73) Proprietor: Centro Nacional de Investigaciones Cardiovasculares Carlos III (F.S.P.), 28029 Madrid (ES); German Cancer Research Center (DKFZ), 69120 Heidelberg (DE)
(72) Inventor: RODRÍGUEZ RAMIRO, Almudena, 28029 MADRID (ES); LORENZO MARTÍN, Cristina, 28029 Madrid (ES); WARDEMANN, Hedda, 69120 Heidelberg (DE); BUSSE, Christian, 69120 Heidelberg (DE)
(74) Representative: Pons IP
(86) International application number: PCT/EP2020/076541
(87) International publication number: WO 2021/058548

(56) References cited:
- WO-A2-2004/057031
- HUI LI ET AL: "Alcohol Metabolism in the Progression of Human Nonalcoholic Steatohepatitis", TOXICOLOGICAL SCIENCES, vol. 164, no. 2, 27 April 2018 (2018-04-27), pages 428 - 438, XP055667420, ISSN: 1096-6080, DOI: 10.1093/toxsci/kfy106
- MICHAEL WIERER ET AL: "Compartment-resolved Proteomic Analysis of Mouse Aorta during Atherosclerotic Plaque Formation Reveals Osteoclast-specific Protein Expression", MOLECULAR & CELLULAR PROTEOMICS, vol. 17, no. 2, 1 February 2018 (2018-02-01), US, pages 321 - 334, XP055667442, ISSN: 1535-9476, DOI: 10.1074/mcp.RA117.000315

## Description

### FIELD OF THE INVENTION

The present invention refers to the medical field. Particularly, the present invention refers to antibodies for the diagnosis and/or treatment of atherosclerosis.

### STATE OF THE ART

Cardiovascular disease, most notably myocardial infarction and stroke, is the first cause of mortality in the world. Atherosclerosis is a chronic inflammatory disease that underlies thrombosis and cardiovascular events, but it can remain asymptomatic for long periods of time. Therefore, it is critical to explore new therapeutic and diagnostic avenues in this area.

In the study Wierer, Michael, et al. "Compartment-resolved proteomic analysis of mouse aorta during atherosclerotic plaque formation reveals osteoclast-specific protein expression." Molecular & Cellular Proteomics 17.2 (2018): 321-334, the authors identified family members of matrix metalloproteinases (Mmps) with both protective and proatherogenic functions to be up-regulated during atherogenesis. The formation of plaques was quantified by single-run, high-resolution mass spectrometry (MS)-based proteomics. Among novel factors in atherosclerosis, they identified matrilin-2, the collagen IV crosslinking enzyme peroxidase in as well as the poorly characterized MAM-domain containing 2 (Mamdc2) protein as being up-regulated in the ECM during atherogenesis.

WO2004057031A2 relates to a method for identifying therapeutic agents for reducing and monitoring the growth, erosion, rupture or stability of an atherosclerotic plaque comprising the analysis of the differential expression of at least two genes coding proteins chosen among Stearoyl CoA desaturase, Phosphatidic acid phosphate, and Phosphoinositide-specific-phospholipase-B1, eventually in association with the analysis of the differential expression of at least one gene coding a protein choosen in the group comprising Aldose reductase and aldehyde reductase, Sphingomyelinase, Acid ceramidase, Ceramide glucosyl transferase, Sphingosin phosphate liase, Thymosine beta 4, Aldehyde dehydrogenase, ATPase Ca++ binding protein and CD163.

Studies in other fields, such as Nonalcoholic Fatty Liver Disease (NAFLD), have demonstrated the importance of some antibodies directed to some ALDHs, such as ALDH4A1. In the study, Li, Hui et al. "Alcohol metabolism in the progression of human nonalcoholic steatohepatitis." Toxicological Sciences 164.2 (2018): 428-438, the authors investigate the alterations in alcohol metabolism processes in response to human NASH progression. Expression and function of ADHs, ALDHs, and catalase were examined in normal, steatosis, NASH (fatty) and NASH (not fatty) human liver samples. Specifically, the study discloses an antibody (sc-39891) directed to ALDH4A1. ALDH4A1 mRNA was significantly decreased in both NASH groups, while no significant changes were observed in the mRNA levels of other alcohol-related enzymes. The decreased ALDH activity in acetaldehyde metabolism in NASH patients may indicate an impaired acetaldehyde detoxification in these patients and increase their sensitivity to alcohol and alcohol associated toxicity. The results provide an indication for making appropriate dosing adjustments for Nonalcoholic Fatty Liver Disease (NAFLD) patients taking drugs that are metabolized by these pathways.

The presence of antibodies in the atherosclerotic plaque was described decades ago, and the connection between autoimmunity and atherosclerosis is well accepted. However, the immunogenic trigger and the impact of the antibody immune response during atherosclerosis are not well understood.

Atherosclerosis causes atheroma plaque formation in the arteries and is the major cause underlying thrombosis, ischemic heart disease and stroke. Retention and subsequent oxidation (ox) of low density lipoprotein (LDL) at the vessel sub-endothelium space is the core trigger of the inflammatory reaction during atherosclerosis. In addition, the adaptive arm of the immune response is known to be critical during atherosclerosis. Antibodies were first detected in atheroma plaques decades ago and distinct functions have been attached to B cells and the antibody immune response during atherosclerosis development. While oxidation-specific epitopes (OSE) such as those contained in oxLDL have been widely studied as immunogenic neoantigens in atherosclerosis, the knowledge of additional antigenic triggers and their impact in disease progression remains very limited.

Therefore, despite of the progress in understanding the atherogenic molecular basis thus opening horizons for several promising novel targets, new and more efficient targeting agents for the diagnosis and treatment of atherosclerosis are in ever-increasing demand.

Therefore, there is an unmet medical need of identifying novel antibodies able to detect atherosclerotic plaque *in vivo* conditions, thus constituting the basis for developing new clinical tools dedicated to molecular imaging and therapy of human atherosclerosis.

The present invention aims to solve the above cited problem by providing antibodies, specifically binding to atherosclerosis lesions *in vivo,* which can be used for the diagnosis or for treating human atherosclerosis.

### DESCRIPTION OF THE INVENTION

### Brief description of the invention

As explained above, the present invention refers to antibodies for the diagnosis and/or treatment of atherosclerosis.

The invention is defined by the appended claims. Any other aspects or embodiments described herein are for illustrative purposes only.

Any references in the description to methods of treatment or diagnosis refer to the compounds, pharmaceutical compositions and medicaments of the present invention for use in a method for treatment of the human (or animal) body by therapy (or for diagnosis).

The inventors of the present invention have performed a high-throughput single cell analysis of the antibody repertoire associated with atherosclerosis. They sequenced more than 1700 antibodies from LDLR-/- mice and control mice and identified 56 antibodies expressed by clones of B lymphocytes expanded *in vivo* in the atherogenic context. Importantly, they found that one third of the expanded antibodies (18 antibodies shown in **Table 1** below) showed reactivity against the atherosclerotic plaque, indicating that a number of antigens in the lesion can trigger an antibody immune response.

On the other hand, by using deep proteomics analysis, it was found that one such antibody, A12, recognizes Aldehyde Dehydrogenase 4 Family Member A1 (ALDH4A1), a mitochondrial dehydrogenase of the proline metabolism. Moreover, it is herein demonstrated that ALDH4A1 distribution is altered during atherosclerosis. Notably, ALDH4A1 levels are increased in the plasma of atherosclerotic mice and of humans with atherosclerosis, supporting the potential use of ALDH4A1 as a biomarker for atherosclerosis. Finally, the present invention shows that treatment of LDLR-/- mice with infusions of A12 delays atherosclerosis progression and reduces circulating free cholesterol and LDL.

Thus, the present invention reveals a new antigenic trigger for the antibody immune response associated with atherosclerosis and opens new avenues for diagnostic and therapeutic interventions in cardiovascular disease.

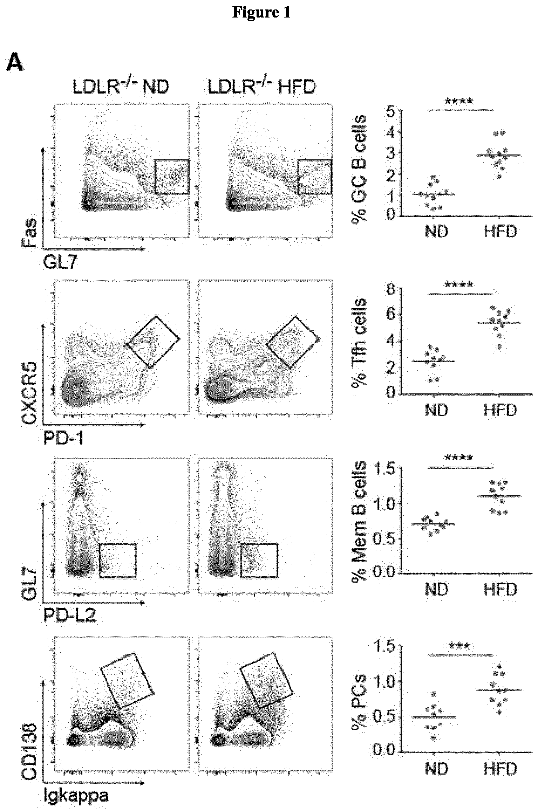

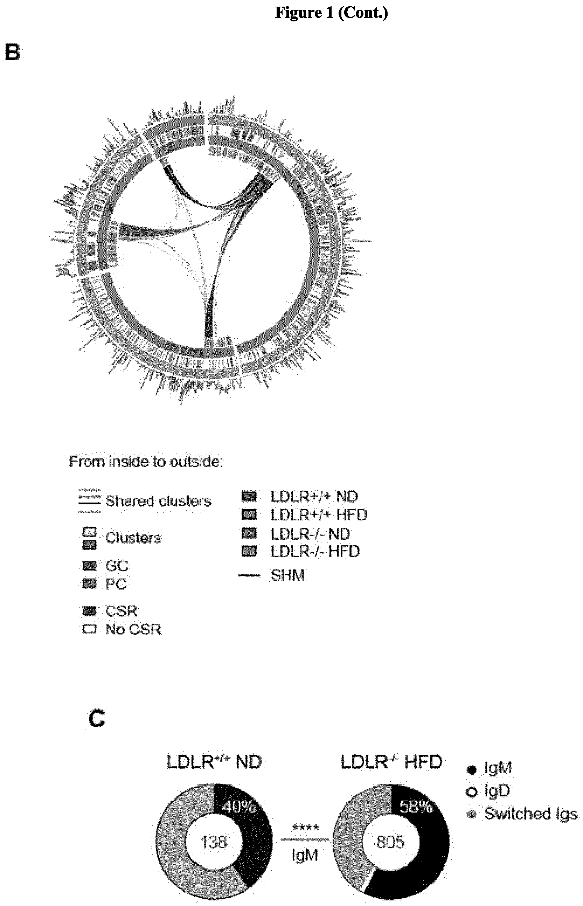

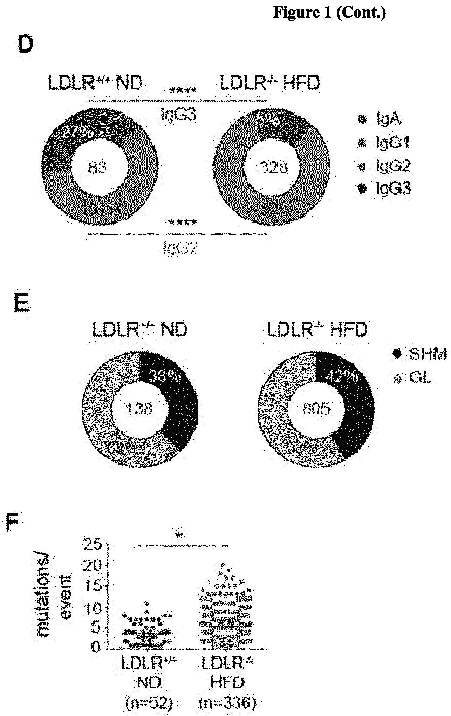

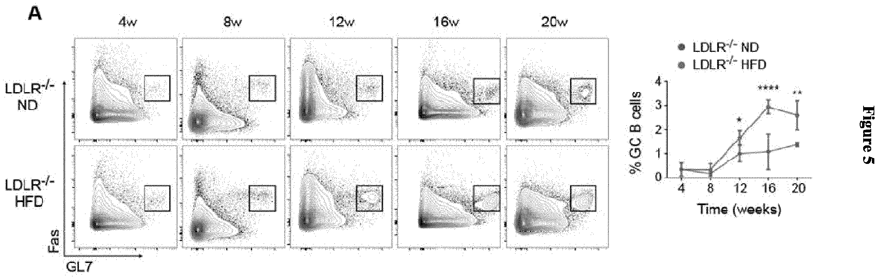

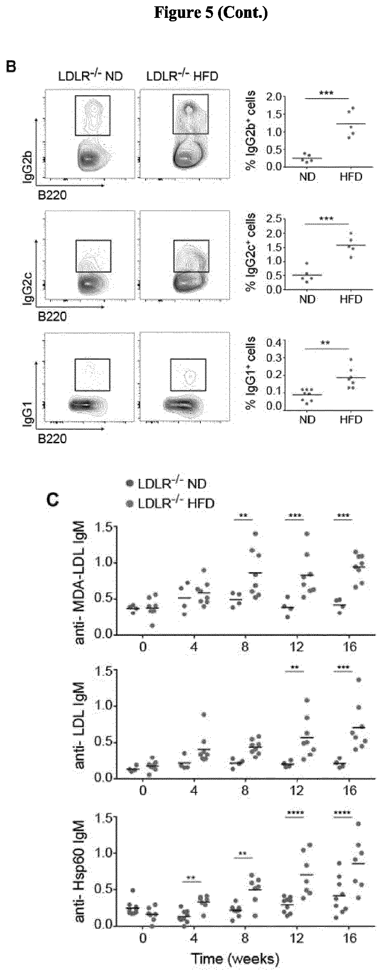

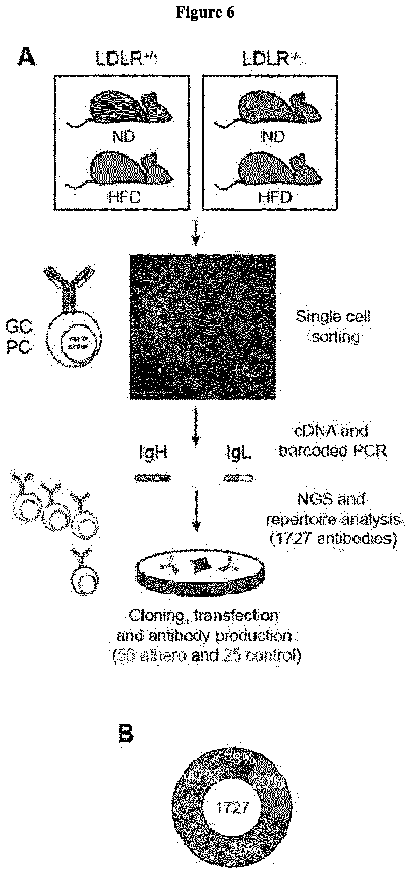

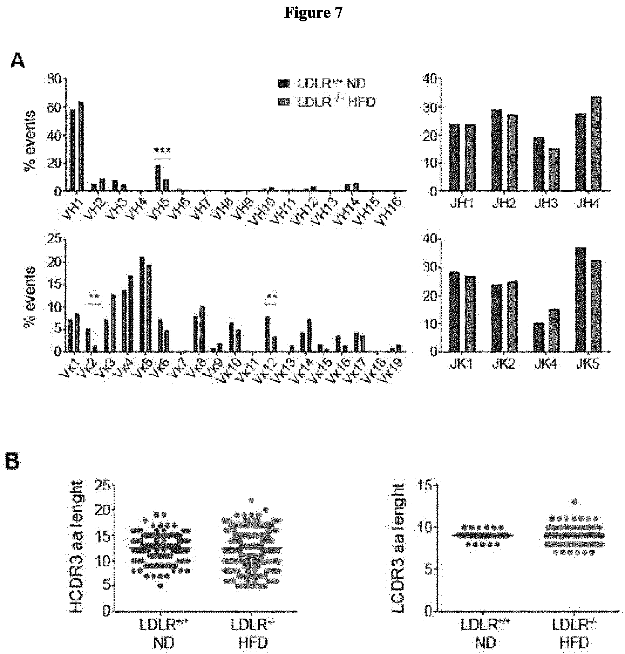

In order to study the antibody immune response associated with atherosclerosis, LDLR-/- mouse model was used, which in combination with high cholesterol and fat diet feeding (HFD), results in atherosclerotic plaque formation. The presence of a germinal center (GC) response was assessed, a critical arm of adaptive immunity, where B cells engage in secondary antibody diversification by class switch recombination (CSR) or somatic hypermutation (SHM) and give rise to memory B cells and high affinity plasma cells (PC). It was found that LDLR-/- HFD mice progressively developed an antibody immune response that involved accumulation of GC B cells (Fas+GL7+), T follicular helper cells (Tfh, CXCR5+PD1+), memory B cells (PD-L2+), IgG+ switched B cells and CD138+kappa+ plasma cells/plasmablasts (PC) **(****Figure 1A****,** **Figure 5A****,** **Figure 5B****).** This response was accompanied by plasma accumulation of IgM antibodies specific for LDL, MDA-LDL and Hsp60 **(****Figure 5C****),** as previously described in different atherosclerotic backgrounds. To analyse the antibody repertoire associated with atherosclerosis, a high-throughput single-cell antibody sequencing of GC B cells and PC from LDLR-/- HFD and control mice were performed **(****Figure 6A****).** Paired sequences for immunoglobulin heavy (IgH) and light (IgL) chains were obtained for more than 1700 antibodies from a total of 17 mice **(****Figure 6B-E**, **Figure 1B****).** Major differences were not found in variable (V) and joining (J) gene usage or in CDR3 length between LDLR-/- HFD and control mice **(****Figure 7****).** However, the distribution of switched antibodies was skewed **(****Figure 1C-D**) and the somatic hypermutation (SHM) load was increased in LDLR-/- HFD mice **(****Figure 1E-F****).** Thus, atherosclerosis progression associates with an antibody immune response and the formation of GCs.

In order to approach the specificity of atherosclerosis-associated antibodies, antibodies from *in vivo* expanded B cells in LDLR-/- HFD mice were first identified, based on identical V(D)J usage of IgH and IgL and identical IgH CDR3 length **(****Figure 6A****).**

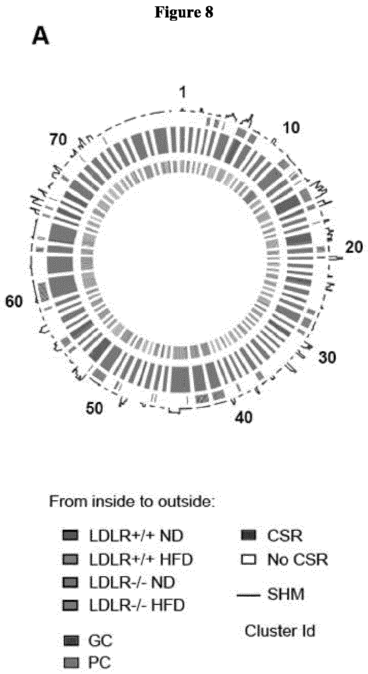

56 such expanded antibodies (clusters) were selected from LDLR-/- HFD mice for cloning and expression **(****Figure 6A****,** **Figure 8A-B**). As controls, 25 antibodies were cloned and expressed from LDLR+/+ ND mice **(****Figure 6A****).**

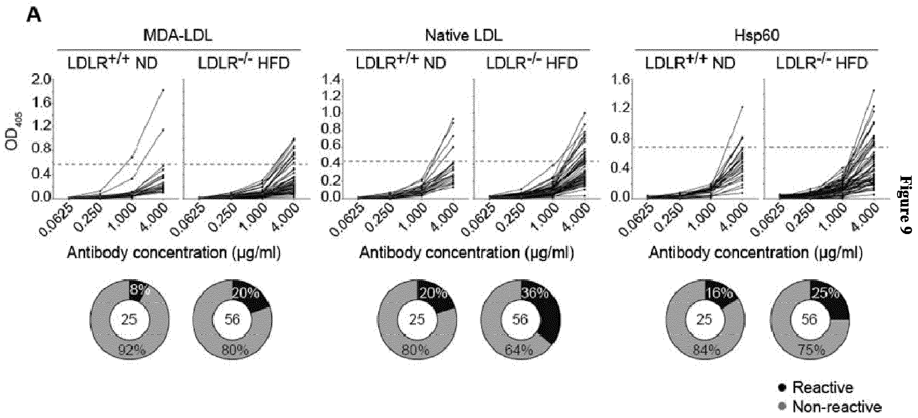

It was found that the fraction of antibodies with specificity for MDA-LDL, native LDL and Hsp60 was slightly, although not significantly, increased in LDLR-/-HFD mice **(****Figure 9A****).**

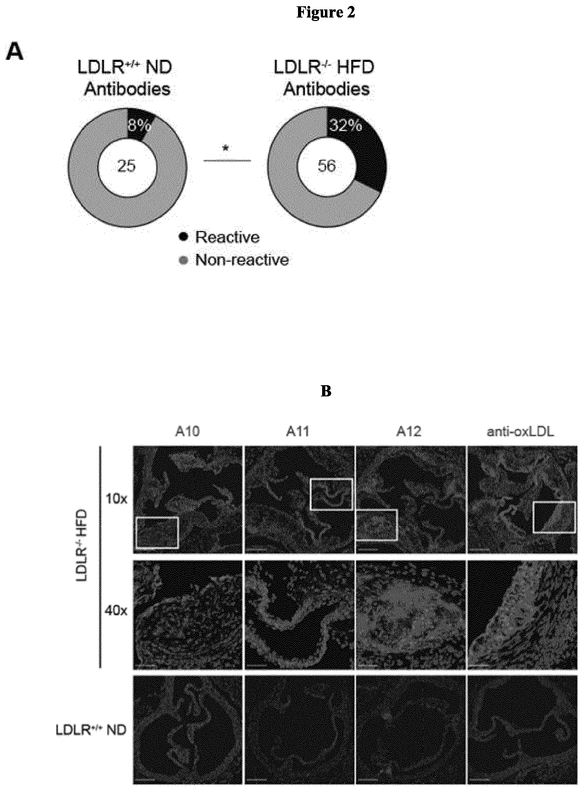

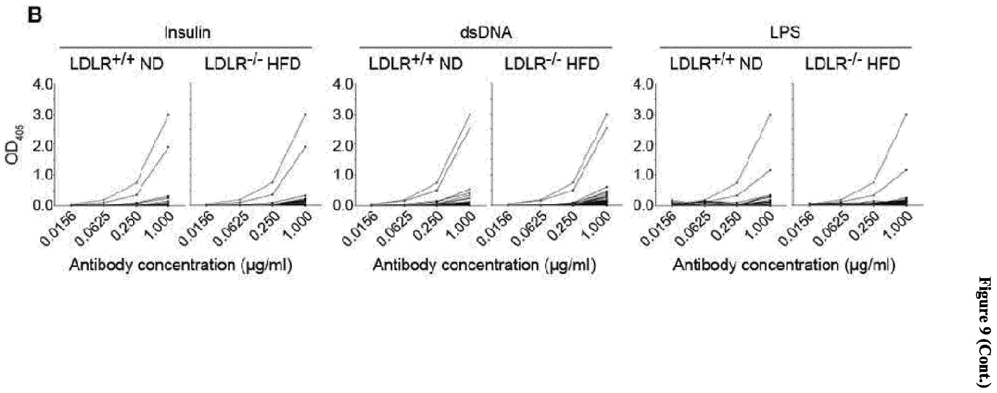

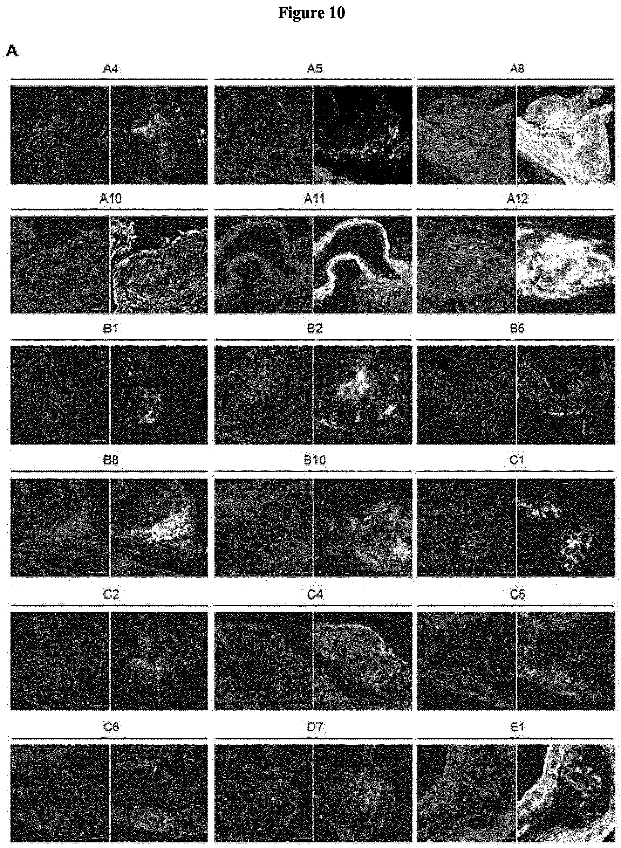

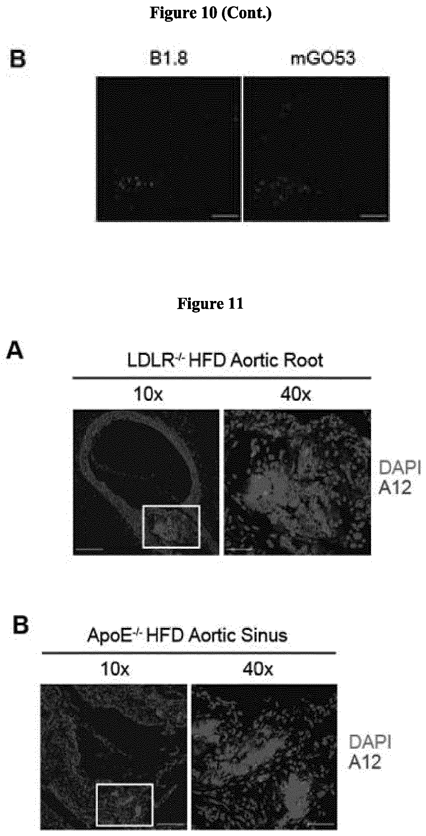

Nevertheless, signs of polyreactivity were not detected in antibodies from either group of mice **(****Figure 9B****).** Then, immunofluorescence staining of aortic sinus sections from atherosclerotic mice was performed. One third of the antibodies cloned from LDLR-/- HFD mice (i.e. the 18 antibodies shown in **Table 1** below) showed reactivity against the atherosclerotic plaque, while only 8% (2) of the antibodies from LDLR+/+ ND control mice did **(****Figure 2A****,** **Figure 10****)..** The pattern of plaque reactivity differed widely among the different self-reactive antibodies and was also distinct from the staining pattern observed with a commercial anti-oxLDL antibody **(****Figure 2B****).** Together, these results indicate that in the context of atherosclerosis, a variety of plaque-associated self-antigens or neoantigens can elicit an antibody immune response.

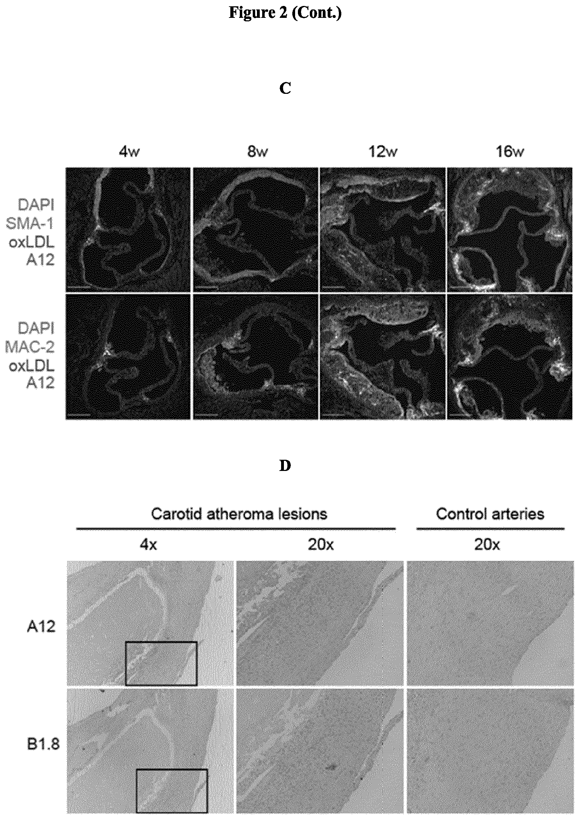

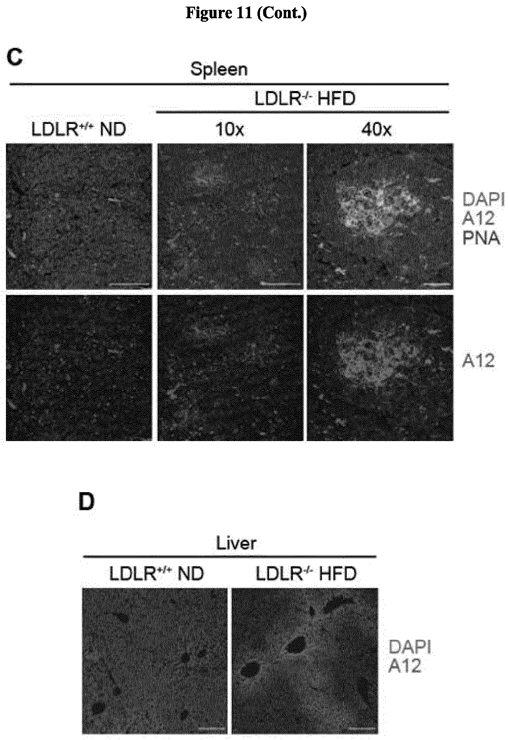

Although all the antibodies listed in **Table 1** showed reactivity against the atherosclerotic plaques, it was decided to focus on A12 antibody in order to gain further insight into the specificity of the antibody response associated to atherosclerosis. A12 antibody showed a strong plaque reactivity in the aortic sinus in LDLR-/- HFD mice **(****Figure 10A****,** **Figure 2B****)** that progressively accumulated during the progression of the disease **(****Figure 2C****).** A12 also stained the aortic root of LDLR-/- HFD mice **(****Figure 11A****),** and aortas from ApoE deficient mice, an alternative mouse model of atherosclerosis **(****Figure 11B****).** It was also detected A12 staining within spleen follicles of LDLR-/- HFD mice **(****Figure 11C****).** This suggests that A12 antigen(s) can circulate to the spleen, and is consistent with A12 being cloned from a spleen B cell. Staining of liver sections revealed that A12 reactivity was enhanced and reshaped around hepatic veins in LDLR-/- HFD atherosclerotic mice, indicating that the distribution of the antigen(s) recognized by A12 is altered in sites other than arteries during atherosclerosis **(****Figure 11D****).** Importantly, it was found that A12 also showed reactivity against human carotid atherosclerotic lesions **(****Figure 2D****).** These data show that A12 recognizes an antigen(s) that is associated with atherosclerosis in different mouse models as well as in the human disease.

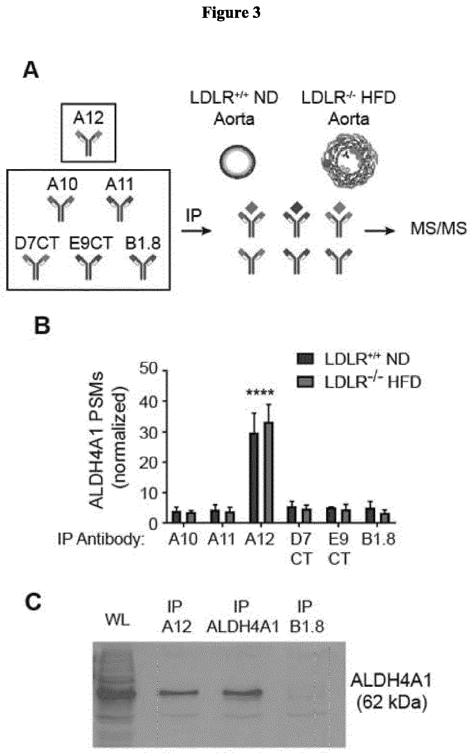

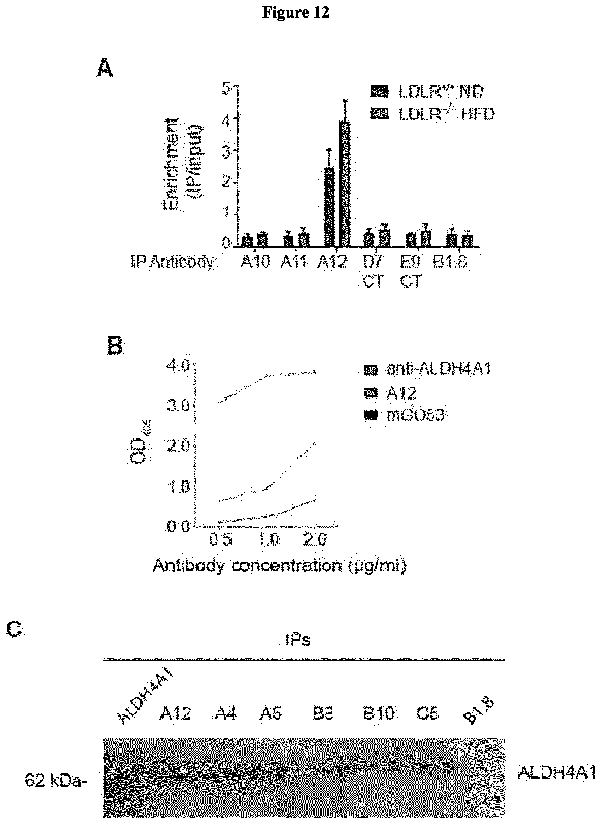

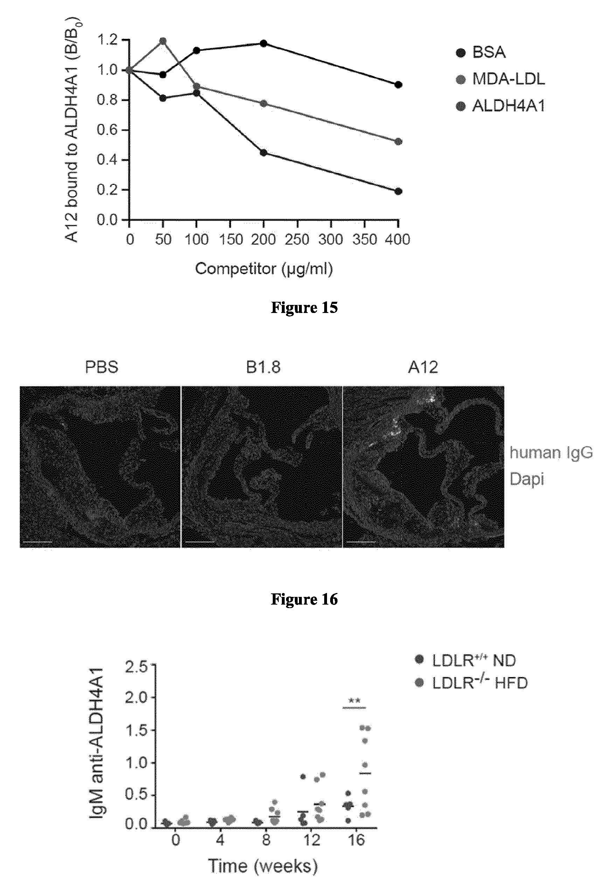

In order to identify the specific antigen(s) recognized by A12, immunoprecipitation of total aorta extracts was performed followed by antigen determination by high-throughput mass spectrometry **(****Figure 3A****).** As controls immunoprecipitates of other antibodies were analysed cloned from LDLR-/- HFD or LDLR+/+ ND control mice, as well as of B1-8, a well characterized antibody specific for 4-Hydroxy-3-nitrophenyl (NP) hapten, and thus unrelated to atherosclerosis. A12 specifically precipitated Aldehyde Dehydrogenase 4 Family Member A1 (ALDH4A1), a mitochondrial dehydrogenase of the proline degradation pathway, while only background counts of ALDH4A1 peptides were obtained from the precipitates of all the control antibodies **(****Figure 3B****,** **Figure 12A****).** The finding that A12 also precipitates ALDH4A1 from wild type aortas suggests that atherosclerosis alters the distribution of this mitochondrial protein and promotes its accumulation in plaque lesions. To confirm binding of A12 to ALDH4A1 immunoprecipitation with blotting were combined using a commercial anti-ALDH4A1 antibody. A12 and anti-ALDH4A1 precipitates were recognized by the anti-ALDH4A1 antibody, while B1-8 precipitate was not **(****Figure 3C****).** Likewise, A12 binding to ALDH4A1 was detected by ELISA **(****Figure 12B****,** **Figure 14****).** Thus, A12 recognizes ALDH4A1 mitochondrial protein.

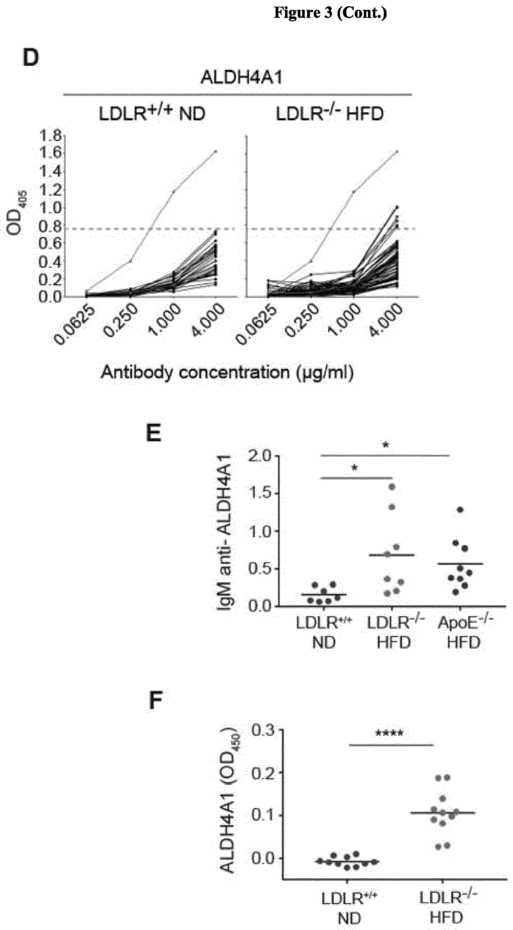

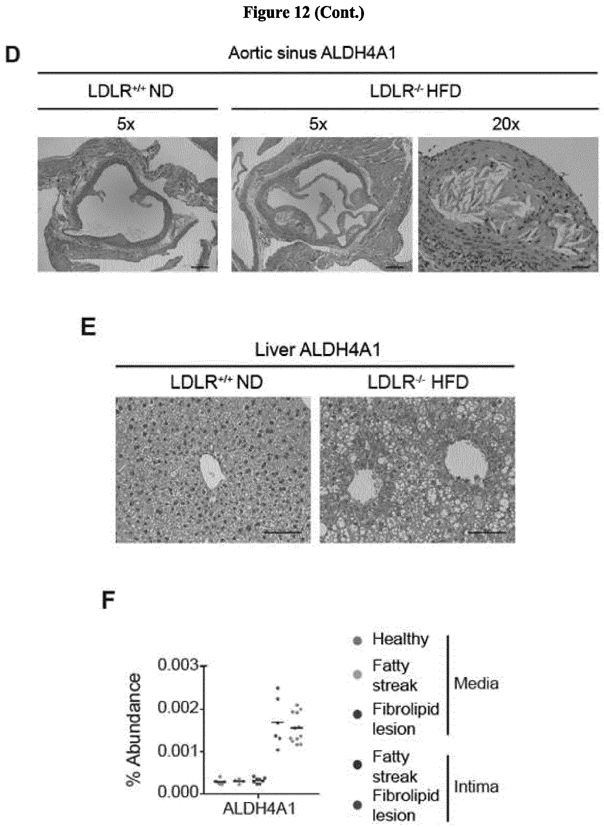

Then, the collection of antibodies cloned in the present invention from LDLR-/- HFD and LDLR+/+ ND mice were screened for ALDH4A1 recognition by ELISA and, in addition to A12, 5 antibodies (B8, C5, A5, B10 and A4) showed ALDH4A1 reactivity and were found in the LDLR-/- HFD group (see **Table 1),** while none was found in the LDLR+/+ ND group **(****Figure 3D****,** **Figure 12C****),** indicating that A12 specificity for ALDH4A1 is not an isolated event during atherosclerosis. This notion was further confirmed in mouse plasma, where it was found higher titters of anti-ALDH4A1 antibodies in LDLR-/- HFD and ApoE-/- atherosclerotic mice than in control mice **(****Figure 3E****,** **Figure 16****).** Staining of aortic sinus sections with commercial anti-ALDH4A1 antibody revealed reactivity against the plaque, similarly to A12 **(****Figure 12D****).** Likewise, anti-ALDH4A1 reactivity in the liver rearranged around hepatic veins in association with atherosclerosis was identified **(****Figure 12E****),** as A12 did **(****Figure 11D****).** In addition, proteomics analysis of human atherosclerotic tissue revealed an increased abundance of ALDH4A1 as compared to normal aortic tissue **(****Figure 12F****).** These results indicate that, in the context of atherosclerosis, ALDH4A1 self-antigen is relocated or accumulated in the aortic plaque and in liver and that it can trigger an antibody immune response.

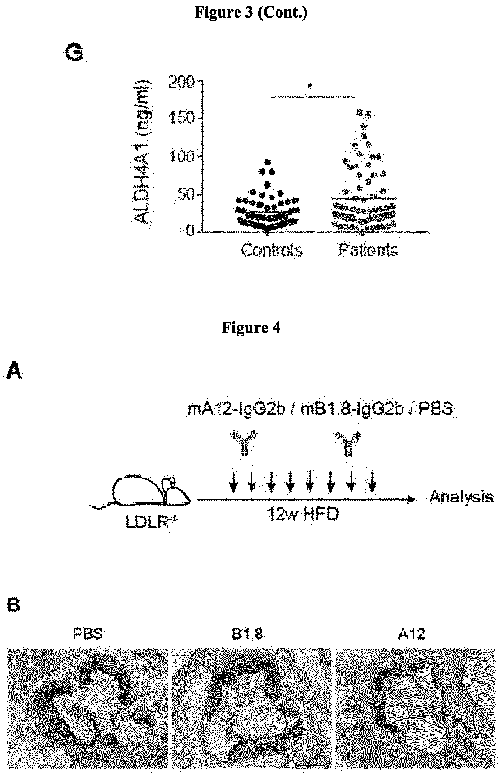

In turn, it was analysed whether ALDH4A1 concentration was also increased in the plasma of atherogenic mice. The present invention demonstrates that, indeed, plasma from LDLR-/- HFD mice has significantly higher levels of ALDH4A1 than controls **(****Figure 3F****).** Moreover, analysis of plasma ALDH4A1 in a cohort of patients with carotid atherosclerosis showed that the levels of ALDH4A1 were significantly higher in the plasma from patients as compared to controls **(****Figure 3G****),** independently of traditional risk factors (see **Table A** showing logistic regression analysis with atherosclerosis presence as dependent variable). Thus, these results strongly suggest that ALDH4A1 could be a valuable novel biomarker for the diagnosis of atherosclerosis.

**Table A**

| | p | O.R. (IC 95%) |
|---|---|---|
| Age | 0.015 | 1.156 (1-029-1.298) |
| Diabetes | 0.304 | 1.929 (0.552-6.741) |
| Smoking | 0.547 | 1.360 (0.500-3.702) |
| HTA | 0.122 | 2.391 (0.792-7.223) |
| CVD | 0.179 | 2.698 (0.635-11.466) |
| Dyslipidemia | 0.045 | 2.890 (1.026-8.142) |
| ALDH4 (per 10 units) | 0.022 | 1.274 (1.035-1.568) |

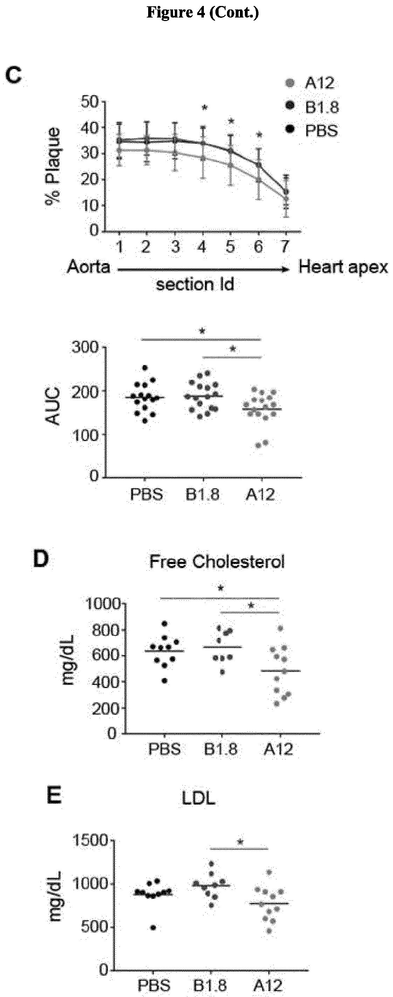

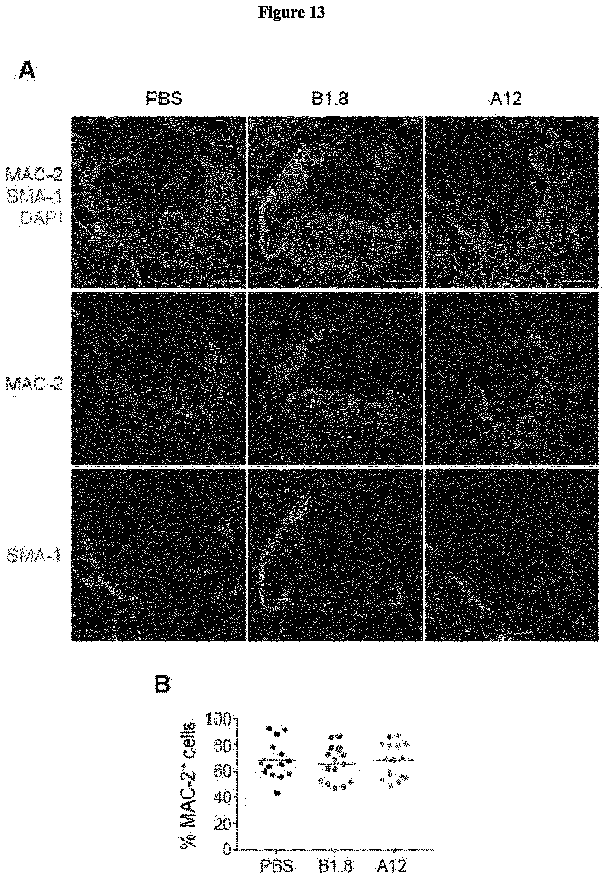

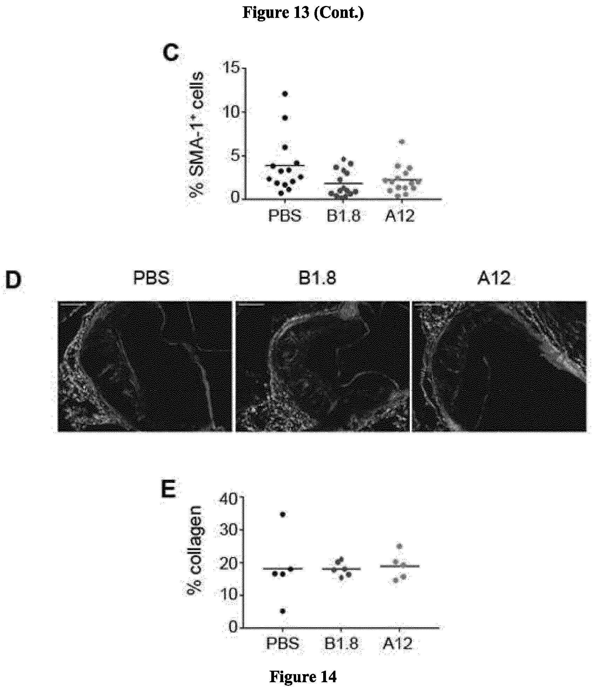

To test whether A12 antibody could have any functional impact on atherosclerosis progression (Figure 15), A12 was cloned and expressed in its original, mouse IgG2b, isotype and performed serial intravenous injections into LDLR-/- HFD mice. As control groups, LDLR-/- HFD mice were injected with PBS or with B1-8 antibody cloned in identical mouse IgG2b frame **(****Figure 4A****).** Aorta analysis after 12 weeks of HFD and antibody treatment revealed a smaller plaque size in mA12-IgG2b treated mice than in mice treated with mB1-8-IgG2b control antibody or with PBS alone **(****Figure 4B****,C).** Although qualitative differences in plaque composition regarding cellular or collagen composition was not detected **(****Figure 13****),** mA12-IgG2b treated mice had decreased levels of plasma free cholesterol and LDL **(****Figure 4D****,E).** These results indicate that A12 infusion delays the progression of atherosclerosis and improves the circulating lipid profile in LDLR-/- HFD mice, suggesting that A12 have a systemic therapeutic effect on atherosclerosis progression.

Thus, the findings provided in the present invention open the way for ALDH4A1 and A12 as very promising clinical tools in cardiovascular disease, both for the diagnosis and for the treatment of atherosclerosis.

Thus, the first embodiment of the present invention refers to antibody or antibody fragment binding ALDH4A1 which comprises HCDR1, HCDR2 and HCDR3 polypeptides and VL comprises LCDR1, LCDR2 and LCDR3 polypeptides and, wherein HCDR1 comprises the sequence SEQ ID NO: 1, HCDR2 comprises the sequence SEQ ID NO: 2, HCDR3 comprises the sequence SEQ ID NO: 3, LCDR1 comprises the sequence SEQ ID NO: 4, LCDR2 comprises the sequence SEQ ID NO: 5 and LCDR3 comprises the sequence SEQ ID NO: 6, for use in a method for *in vivo* imaging of atherosclerosis lesions in a patient. Preferably, for use in a method for *in vivo* diagnosis of atherosclerosis

**Table 1** includes the six complementarity-determining regions (CDR) of the variable region corresponding to the antibody:

**Table 1**

| **Ab** | **Igh_cdr1** | **Igh_cdr2** | **Igh_cdr3** | **Igk_cdr1** | **Igk_cdr2** | **Igk_cdr3** |
|---|---|---|---|---|---|---|
| **A12*** | SEQ ID NO: 1 | SEQ ID NO: 2 | SEQ ID NO: 3 | SEQ ID NO: 4 | SEQ ID NO: 5 | SEQ ID NO: 6 |

| | | | | | | |
|---|---|---|---|---|---|---|
| *Ab (antibody). IGH (heavy chain). IGK (light chain). *Antibodies showing ALDH4A1 reactivity.* | | | | | | |

The complete sequence of the variable region corresponding to the heavy and light chain of the antibody is included in **Table 2:**

**Table 2**

| **Ab** | **Variable region Igh** | **Variable region Igk** |
|---|---|---|
| **A12*** | SEQ ID NO: 109 | SEQ ID NO: 110 |

| | | |
|---|---|---|
| *Ab (antibody). IGH (heavy chain). IGK (light chain). *Antibodies showing ALDH4A1 reactivity.* | | |

More specifically, please find below the aminoacid sequence of the variable region corresponding to the heavy and light chain of theantibody, wherein the aminoacid sequence corresponding to the CDR1, CDR2 and CDR3 are underlined.
**A12 IgH (SEQ ID NO: 109)** 
E V Q L V E S G G D L V K P G G S L K L S C A A S G F T F S S Y G M
S W V R Q T P D K R L E W V A T I S S G G S Y T Y Y P D S V K G R F
T I S R D N A K N T L Y L Q M S S L K S E D T A M Y Y C A R P H V V
T T P G W Y F D V W G T G T T V T V S S
**A12 IgK (SEQ ID NO: 110)**

D I V M T Q S P A T L S V T P G D R V S L S C R A S Q S I S D Y L H
W Y Q Q K S H E S P R L L I K Y A S Q S I S G I P S R F S G S G S G S
D F T L S I N S V E P E D V G V Y Y C Q N G H S F P L T F G A G T K
L E L K

In a preferred embodiment, the present invention refers to the antibody A12 characterized in that the heavy chain variable region (VH) comprises the SEQ ID NO: 115 and the light chain variable region (VL) comprises the SEQ ID NO: 116.

In a preferred embodiment, the method for in vivo diagnosis of atherosclerosis comprises the visualization of the atherosclerosis lesions by detection of the antibody previously administered to the patient. In a preferred embodiment, the method comprises the following steps: a) intravenous injection of the antibody for *in vivo* imaging into the patient, and b) visualization of the atherosclerosis lesions by detection of the antibody of step a) in the patient.

For the purpose of the present invention the following definitions are included:
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.

### Description of the figures

**Figure 1****. The antibody immune response during atherosclerosis. (A)** LDLR-/- pro-atherogenic mice (n=9-11) were fed with either normal diet (ND) or high fat diet (HFD) for 16 weeks. Representative flow cytometry plots and their quantification are shown for spleen germinal center (GC) B cells (Fas⁺GL7⁺, gated on B220⁺), T follicular helper (Tfh) cells (CXCR5⁺PD-1⁺, gated on CD4⁺), memory B cells (PD-L2⁺, gated on B220⁺) and plasma cells (PCs) (CD138⁺Igκ⁺). Statistical analysis was done with two-tailed unpaired Student's *t*-test. ***P<001 and ****P<0.0001. **(B)** Circos plot representation of the molecular features of the 1727 antibodies sequenced from LDLR+/+ ND mice (n= 138 antibodies), LDLR+/+ HFD (n=347 antibodies), LDLR-/- ND (n= 437 antibodies), LDLR-/- HFD (n=805 antibodies). Cell of origin (GC or PC), CSR and SHM as well as antibody clusters, are indicated. **(C)** Proportion of IgM, IgD and class-switched immunoglobulins in each group. **(D)** IgA, IgG1, IgG2 and IgG3 isotype subclass distribution within class-switched events. Statistical analysis was done with Fisher's exact test. ****P<0.0001. **(E)** Proportion of mutated (SHM) and germ line (GL) antibodies in each group. **(F)** Number of somatic mutations at IgH within mutated antibodies. Statistical analysis was done with Mann-Whitney test. *P≤0.05.
**Figure 2****. Antibodies cloned from atherosclerotic mice show plaque reactivity.** Atheroma plaque reactivity was tested by immunofluorescence in aortic sinus cryosections from LDLR-/- mice fed with HFD during 12 weeks. **(A)** Proportion of antibodies showing atheroma plaque reactivity (2/25 LDR+/+ ND and 18/56 LDLR-/- HFD) Statistical analysis was done with Fisher's exact test. *P≤0.05.**(B)** Immunofluorescence staining of aortic sinus cryosections from LDLR-/- HFD mice and wild type mice with representative reactive antibodies A10, A11 and A12 cloned from LDLR-/- HFD mice (10x and 40x magnification). Staining with an anti-oxLDL antibody is shown on the right. **(C)** Inmunofluorescence staining of aortic sinus from LDLR-/- HFD mice with A12 antibody (white), anti-oxLDL (red), DAPI (blue) and anti-SMA-1 (top, green) or anti-MAC-2 (bottom, green). Time of HFD feeding is indicated above. **(D)** Paraffin-embedded sections of human carotid atherosclerotic lesions and control arteries were stained with A12 or with B1-8 anti-NP antibody, by immunohistochemistry (4x and 20x magnification). **(B-C)** Scale bar 10x= 200 µm; 40x= 50 µm.
**Figure 3****. ALDH4A1 mitochondrial self-antigen is recognized by A12 antibody and can be used as an atherosclerosis biomarker. (A)** Experimental approach to determine the specificity of A12 antibody. Aortas from LDLR+/+ ND and LDLR-/- HFD mice were lysed and immunoprecipitated (IP) with A12 antibody, followed by tandem Mass Spectrometry (MS/MS) to identify bound proteins. A10 and A11 antibodies from LDLR-/-HFD mice, D7CT, E9CT antibodies from LDLR+/+ ND mice and anti-NP B1-8 antibody were used as controls. **(B)** Number of normalized Peptide Spectrum Matches (PSMs) identified for ALDH4A1 protein in each IP. (Student t-test, ****p < 0.0001). n=3 independent experiments. **(C)** Validation of A12 specificity for ALDH4A1 by IP combined with western blot analysis. LDLR-/- HFD total aorta lysate was IP-ed with either A12, commercial anti-ALDH4A1 antibody or anti-NP B1-8 antibody. Precipitates or whole lysate (WL) were then blotted with anti-ALDH4A1 antibody. Expected ALDH4A1 size is indicated (62 KDa). **(D)** Antibodies cloned from control (n=25) and LDLR-/- HFD (n=56) mice were tested by ELISA for their reactivity against ALDH4A1. Graph shows OD₄₀₅ values at the indicated antibody concentrations. Red lines represent the non-reactive negative control antibody mGO53 ¹. Blue lines represent commercial anti-ALDH4A antibody. **(E)** Quantification of anti-ALDH4A1 IgM antibodies in plasma from LDLR+/+ ND (n=7), LDLR-/- HFD (n=8) and ApoE-/- HFD (n=9) mice determined by ELISA. **(F)** Quantification of ALDH4A1 protein levels in plasma from LDLR+/+ ND (n=10) and LDLR-/- HFD (n=11) mice determined by ELISA. Statistical analysis was done with two-tailed unpaired Student's t-test or one-way ANOVA when appropriate. *q≤0.05, ****P<0.0001. **(G)** Quantification of ALDH4A1 protein levels in plasma from human control individuals (n=54) and carotid atherosclerosis patients (n=63) determined by ELISA. Statistical analysis was done with Mann-Whitney test. *P≤0.05.
**Figure 4****. A12 infusion delays atherosclerosis progression in LDLR-/-HFD mice. (A)** Experimental approach. A12 and B1-8 IgH were cloned into an expression vector containing the mouse IgG2b constant region, the original isotype of A12 as found in LDLR-/-HFD atherosclerotic mice (mA12-IgG2b, mB1-8-IgG2b). LDLR-/- mice were injected intravenously with 100□g of mA12-IgG2b (n=16 mice) or mB1.8-IgG2b (n=16 mice) or with PBS (n=15 mice) every 10 days for 12 weeks and fed with HFD throughout. Mouse number is the sum of two independent experiments. **(B)** Representative Oil-Red staining of aortic sinus cryosections of LDLR-/- HFD mice treated with PBS, B1.8 and A12. **(C)** Upper graph, quantification of atheroma plaque in the aortic sinus (7 sections were quantified from each mice, from the aorta to the heart apex). The proportion of plaque in each section is shown as quantified by Image J. Lower graph, quantifications shown in upper panel are plotted as Area Under the Curve (AUC). **(D)** Quantification of free cholesterol and **(E)** LDL levels in plasma from LDLR-/- HFD mice treated with PBS, B1.8 or A12. Statistical analysis was done with one-way or two-way ANOVA. *q≤0.05.
**Figure 5****. Germinal center response and antibody production during atherosclerosis.** LDLR-/- pro-atherogenic mice were fed with either normal diet (ND) or high fat diet (HFD) for 4, 8, 12, 16 or 20 weeks and the GC response in the spleen was assessed by flow cytometry. Representative plots and their quantification are shown for **(A)** Germinal center (GC) B cells (Fas⁺GL7⁺, gated on B220⁺) at the different time points and **(B)** B cells expressing class-switched immunoglobulins (IgG2b, IgG2c and IgG1) after 16 weeks of ND/HFD treatment. **(C)** Plasma from LDLR-/- mice fed with ND (n=4-9) or HFD (n=8) was collected at different time points (0, 4, 8, 12, 16 weeks) and IgM antibody titers specific for MDA-LDL, LDL and Hsp60 were determined by ELISA. Data from relative absorbance at 450 nm is shown. Statistical analysis was done with two-tailed unpaired Student's t-test or two-way ANOVA.*P≤0.05, **P<0.01, ***P<0.001 and ****P<0.0001.

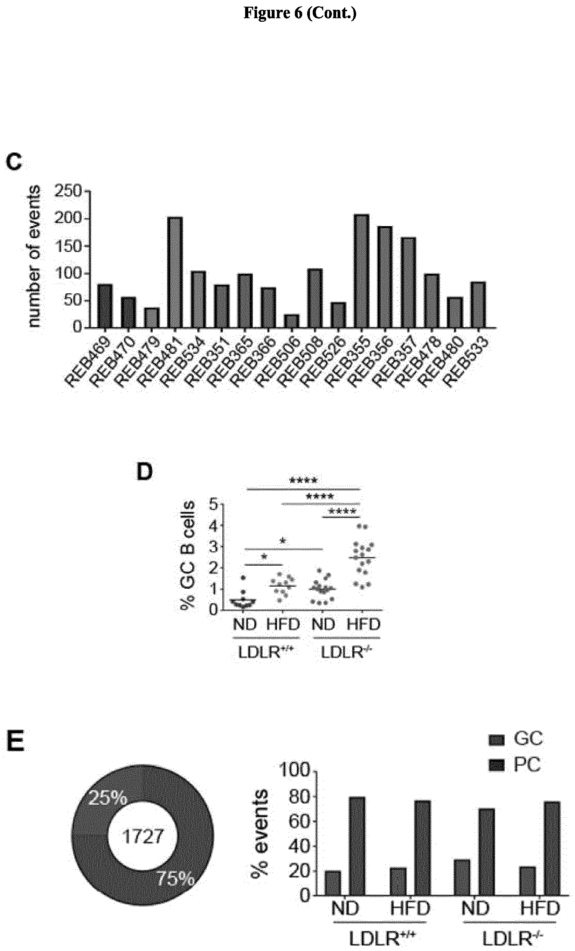

**Figure 6****. Identification of atherosclerosis-associated antibodies by single cell sequencing and cloning of immunoglobulin genes. (A)** Schematic overview of the cell isolation and antibody expression-cloning strategy. Individual spleen GC B cells and plasma cells (PC) from LDLR+/+ ND (n=2, purple), LDLR+/+ HFD (n=3, orange), LDLR-/- ND (n=6, grey) and LDLR-/- HFD (n=6, green) were isolated by single cell sorting. IgH and IgL cDNAs from sorted cells were then PCRed and sequenced. Successful paired (IgH + IgL) sequences were obtained for a total of 1727 individual cells. 56 antibodies representative of expanded B cell clones (clusters) from atherogenic mice (LDLR-/- HFD) were cloned into expression vectors containing the human IgG1 constant region for IgH and the kappa constant region for IgL, as described ². Expression vectors were transfected in eukaryotic cells and antibodies were harvested from supernatants for further analysis. As controls, 25 antibodies from LDLR+/+ ND mice were cloned and expressed in eukaryotic cells. **(B)** Distribution of the 1727 events in the four groups of mice and **(C)** number of events per mouse. **(D)** Proportion of GC B cells (Fas⁺GL7⁺, gated on B220⁺) in spleen of the same four groups of mice analyzed by flow cytometry. **(E)** Global proportion of GC B cells and PCs in the sequenced events (left) and proportion of GC and PC in each group of mice (right). Statistical analysis was done with one-way ANOVA. *q≤0.05, ****q<0.0001.
**Figure 7****. Ig gene analysis of GC B cell and PC antibodies from LDLR+/+ ND and LDLR-/- HFD mice.** Data summarize the IgH and IgL gene sequence analysis from single GC B cells and PC from LDLR+/+ ND (138 antibodies) and LDLR-/- HFD (805 antibodies) mice. (A) IgH and IgL V gene family and J gene usage. **(B)** IgH and IgL CDR3 amino acid length. Statistical analysis was done with Fisher's exact test. **P<0.01, ***P<0.001.

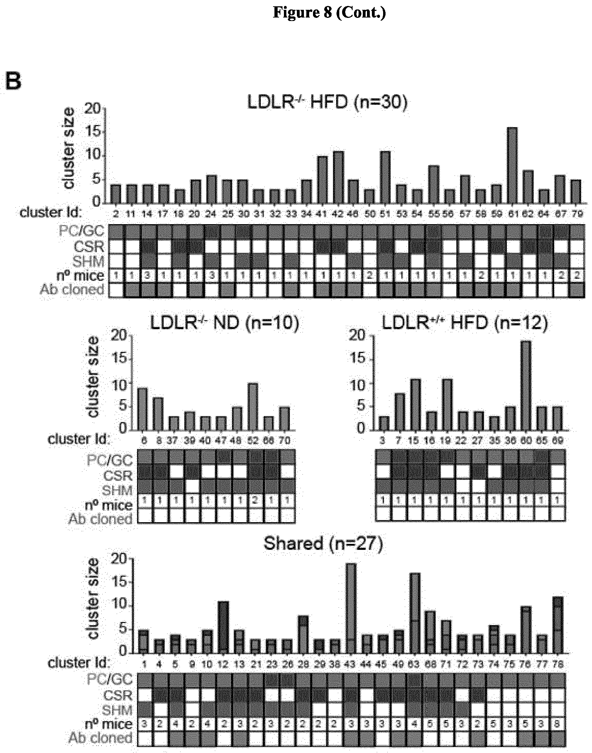

**Figure 8****. Analysis of expanded clones (clusters) within sequenced antibodies. (A)** Circos plot representation of the molecular features of the identified expanded clones. **(B)** Cluster summary. Bars show cluster size (number of antibodies). Checked boxes underneath indicate the population or origin (PC/GC), the presence of CSR, SHM, and the number of mice sharing the cluster. Green-checked boxes indicate the antibodies cloned for subsequent analysis.
**Figure 9****. Reactivity of antibodies cloned from LDLR-/-HFD mice. (A)** A fraction of the antibodies cloned from LDLR-/-HFD mice show reactivity against MDA-LDL, LDL or Hsp60. Antibodies cloned from control (n=25) and atherogenic (n=56) mice were tested by ELISA for their reactivity against MDA-LDL, native LDL and Hsp60. Graph shows OD₄₀₅ values at 4 µg/ml antibody concentration and three consecutive 1:4 dilutions. Red lines represent the non-reactive negative control antibody mGO53. The proportion of antibodies showing reactivity is shown below each graph. **(B)** Antibodies cloned from LDLR-/-HFD mice are not polyreactive. Antibodies were tested for polyreactivity with insulin, dsDNA and LPS. Highly polyreactive ED38 antibody (green lines), low-polyreactive JB40 antibody (purple lines) and non-polyreactive mGO53 antibody (red lines) are shown as controls.
**Figure 10****. Plaque reactivity of the antibodies cloned from LDLR-/-HFD mice. (A)** Representative staining of the 18 positive antibodies from LDLR-/- HFD mice in aortic sinus cryosections from LDLR-/- HFD mice. Merge image of antibody staining (red) and Dapi (blue) is shown in the left and image showing only antibody signal (white) is shown in the right for each antibody. 40x magnification; scale bar= 50 µm. **(B)** B1.8 and mGO53 negative controls antibodies used for classifying the antibodies into reactive or not reactive. 10x magnification; scale bar= 200 µm.
**Figure 11****. Analysis of A12 reactivity by immunofluorescence. (A)** Representative staining of A12 antibody (red) in cryosections from LDLR-/- HFD aortic root. **(B)** Representative staining of A12 antibody (red) in cryosections from ApoE-/- HFD aortic sinus. **(C)** A12 staining (green) of the spleen sections from wild type and LDLR-/- HFD mice, co-stained with PNA (red). **(D)** A12 staining (green) of liver from wild type and LDLR-/- HFD mice. Scale bar 10x= 200 µm; 40x= 50 µm.
**Figure 12****. ALDH4A1 mitochondrial self-antigen is recognized by A12 antibody and associates with atherosclerosis in both mice and humans. (A)** Enrichment of ALDH4A1 in each immunoprecipitation (PSMs IP/PSMs input). **(B)** Validation of A12 specificity for ALDH4A1 by ELISA. Plates were coated with ALDH4A1-FLAG protein and incubated with either mGO53 antibody ¹ as a negative control, A12 or anti-ALDH4A1 as a positive control. Data from relative absorbance at 405 nm is shown. **(C)** Validation of athero antibodies specificity for ALDH4A1 by IP combined with western blot analysis. HEK293T cells transfected with ALDH4A1-FLAG lysates were IP-ed with either A12, A4, A5, B8, B10, C5 athero antibodies, commercial anti-ALDH4A1 antibody or B1.8 anti-NP irrelevant antibody. Precipitates were then blotted with anti-ALDH4A1 antibody. Expected size for ALH4A1 is indicated (62 KDa). **(D)** Representative immunohistochemistry staining of anti-ALDH4A1 antibody in aortic sinus sections of wild-type or LDLR-/- HFD mice. **(E)** ALDH4A1 staining in liver sections of wild-type and LDLR-/- HFD mice. **(F)** Quantification of ALDH4A1 protein abundance in the media and intima layer of different human samples (healthy, fatty streak lesion, and fibrolipid lesion).
**Figure 13****. Evaluation of atheroma plaque composition after A12 antibody treatment. (A)** Representative immunofluorescence images of aortic sinus cryosections of LDLR-/- HFD mice treated with PBS, B1.8 or A12 stained with MAC-2 (red) and SMA-1 (green). **(B)** Quantification of the proportion of plaque with macrophages (MAC-2⁺). (C) Quantification of the proportion of plaque with smooth muscle cells (SMA-1⁺). **(D)** Picrosirius Red staining of aortic sinus cryosections of LDLR-/- HFD mice treated with PBS, B1.8 or A12. **(E)** Quantification of plaque collagen content.
**Figure 14****. Characterization of A12 antibody specificity by competition immunoassays. A12** antibody (1 µg/ml) was preincubated with increasing concentrations (0, 50, 100, 200, 400 µg/ml) of the indicated competitors (BSA, MDA-LDL or ALDH4A1-FLAG) overnight at 4 °C. A12-competitor solutions were added to ALDH4A1-FLAG protein (5 µg/ml) coated ELISA plates, and bound A12 antibody was detected. Results are expressed as ratios of A12 binding to ALDH4A1-FLAG in the presence (B) or absence (B0) of the competitor. These results distinguish ALDH4A1 from MDA-LDL A12 reactivity and indicate that A12 has a greater specificity for ALDH4A1 than for MDA-LDL.
**Figure 15****. A12 antibody reaches the atherosclerotic plaque *in vivo.*** 10-week old LDLR-/- mice were fed with HFD for 12 weeks, infused with PBS, huB18-IgG1 antibody or huA12-IgG1 antibody and sacrificed 3 days later. Representative immunofluorescence stainings of aortic sinus cryosections with anti-human IgG antibody are shown (scale bar= 200 µm). We found that aortas of huA12-IgG1 infused mice showed a staining pattern very similar to that previously observed in *ex vivo* A12 stainings. Thus, this experiment shows that A12 antibody can reach the atherosclerotic plaque *in vivo.*
**Figure 16****. Anti-ALDH4A1 antibodies accumulate during atherosclerosis progression.** ELISA determination of anti-ALDH4A1 IgM antibodies in plasma from LDLR+/+ ND mice (purple, n=5) and LDLR-/- HFD mice (green, n=8) at the indicated times of ND or HFD feeding. **q<0.01; two-way ANOVA.

### Detailed description of the invention

### Example 1.1. Mice.

Male low-density lipoprotein-receptor deficient (LDLR-/-) mice were originally obtained from Jackson Laboratories (002207) and fed with a high-fat diet (HFD) containing 22.1% crude fat and 0.2% cholesterol (Ssniff Spezialdiäten) for 4 to 20 weeks when indicated. LDLR+/+ C57BL/6 wild-type mice were originally obtained from Envigo (C57BL/6JOlaHsd). For several experiments, Apolipoprotein E deficient (ApoE-/-) pro-atherogenic mice were also used, originally obtained from Charles River (B6.129P2-Apoetm1Unc/J), and fed with the same HFD described above. All animals were housed in specific pathogen-free conditions, under a 12 h dark/light cycle with food and water ad libitum. Animal procedures were approved by the CNIC Ethics Committee and the Madrid regional authorities (PROEX 377/15) and conformed to EU Directive 2010/63/EU and Recommendation 2007/526/EC regarding the protection of animals used for experimental and other scientific purposes, enforced in Spanish law under Real Decreto 1201/2005.

### Example 1.2. Human Samples.

Plasma was obtained from 63 patients undergoing carotid endarterectomy (carotid stenosis>70%) in IIS-Fundacion Jimenez Diaz and Hospital de Galdakao. From these 63 patients, cardiovascular risk factors were assessed for 56 of them, which were included in the logistic regression analysis (see **Table B** showing the clinical characteristics of the participants).

**Table B**

| | Control (n=54) | Atherosclerosis (n=56) |
|---|---|---|
| Age, years | 65±0.2 | 70±7.7 |
| Gender (M/F) | 54/0 | 49/7 |
| Smoker (Yes/no) | 30/24 | 20/36 |
| Diabetes (Yes/no) | 7/47 | 25/31 |
| HTA (Yes/no) | 30/24 | 46/10 |
| CVD (Yes/no) | 5/49 | 18/38 |
| Dyslipidemia (Yes/no) | 17/37 | 42/14 |

The presence of cardiovascular risk factors includes: diabetes mellitus, arterial hypertension, dyslipidemia and smoking habits. Patients were considered as diabetics if they were under treatment (supervised diet, hypoglycaemic oral medication, insulin) or a basal glycemia >120 mg/dL and/or HbAlc >=6.5%. We defined hypertension as systolic blood pressure (sBP) >140 mmHg and/or diastolic pressure (dBP) >=90 mmHg measured during the examination (after the participant had been sitting for at least 30 minutes) or if the participant was already taking hypotensive medication. Dyslipidemia was diagnosed as the presence of at least 1 of the following characteristics: total cholesterol >200 mg/dL, LDL-cholesterol >130 mg/dL, or tryglicerides >200 mg/dL or the participant was already taking statins or fibrates medication. Smoking was defined as "current smokers" or "nonsmokers" (including "ex-smokers", those who stopped smoking at least 6 months before the inclusion in the study), with a smoker defined by the history of smoking (≥ 10 cigarettes per day > 1 year). In some cases, carotid atherosclerotic plaques obtained after endarterectomy were kept in paraformaldehyde for further immunohistological analysis.

Moreover, plasma was obtained from 54 controls with no stenosis in the carotid artery that were recruited from a screening program for both carotid atherosclerosis and abdominal aortic aneurysm between 65 years-old men. The absence of vascular disease was confirmed with a physical examination and an ultrasound scan.

The Ethical committee on human research at IIS-Fundacion Jimenez Diaz-Autonoma University approved the study, which was performed in accordance with the principles outlined in the Declaration of Helsinki, and all participants gave written informed consent.

In addition, aortic tissue samples from the media and intima layers were obtained from dead organ donors with the authorization of the French Biomedicine Agency (PFS 09-007, BRIF BB-0033-00029; AoS BBMRI-EU /infrastructure BIOBANQUE ; No. Access : 2, Last : April 15, 2014. [BIORESOURCE]). Some of these samples were macroscopically normal (H) and devoid of early atheromatous lesions and were used as healthy controls (9 individuals) for comparison with those with samples with fatty streaks (FS) (7 individuals for media layer and 6 in the case of the intima layer) or fibrolipidic (FL) plaques (11 individuals in the case of media layer and 12 in the case of intima layer).

### Example 1.3. Flow Cytometry.

Single-cell suspensions were obtained from spleen, blocked with anti-mouse CD16/CD32 antibodies and stained with fluorophore or biotin-conjugated anti-mouse antibodies (BD Pharmingen or Biolegend) to detect B220 (RA3-6B2), Fas (Jo2), GL7, CD4 (RMY-5), CXCR5 (L138D7), PD-1 (29F-1A12), PD-L2 (TY25), CD138 (281-2), Igkappa (187.1), IgG2b (RMG2b-1), IgG2c (polyclonal) and IgG1 (A85-1). Streptavidin (BD) was used in the case of biotin-conjugated antibodies. Only live lymphocytes were analyzed. For Ig kappa intracellular staining, cells were fixated and permeabilized using the Foxp3/Transcription Factor Staining Buffer Set (ThermoFisher Scientific). Samples were acquired on LSRFortessa or FACSCanto instruments (BD Biosciences) and analyzed with FlowJo V10.4.2 software.

### Example 1.4. Immunoglobulins Detection in Plasma by ELISA.

For determination of plasma-specific IgM titers against prototypic atherosclerosis-associated antigens, mouse IgM ELISA Quantitation Kit (Bethyl laboratoires) was used in accordance with the manufacturer's instructions. In brief, plates were coated with MDA-LDL (3 µg/ml, Holzel), native LDL (3 µg/ml, Holzel), Hsp60 (1 µg/ml, Enzo) or ALDH4A1-FLAG (5 µg/ml); blocked with 1% BSA and incubated with mouse plasmas of different time points (1/50 dilution). A goat anti-mouse IgM detection antibody conjugated to HRP followed by TMB substrate solution was added to the plate and finally measure the optical density (OD) at 450 nm.

### Example 1.5. Single-Cell Sorting and Sequencing of Immunoglobulin Transcripts.

Single-cell sorting into 384-well plates and Matrix single-cell PCR and sequencing were performed as described before. In brief, germinal center B cells (Fas⁺GL7⁺B220⁺) and plasma cells (CD138⁺B220⁻) were isolated from spleen and sorted into lysis buffer-containing wells on an Aria III (BD Biosciences) using single-cell mask settings and FSC-H/FSC-W-based doublet detection. cDNA of the individual cells was generated using random hexamer primers. *Igh, Igk* and *Igl* transcripts were amplified using a semi-nested PCR strategy, which in parallel incorporated dual barcodes into the resulting amplicons. To increase the efficiencies for non-class-switched cells, two additional *Ighd* primers were added to the *Igh* PCR master mix in the first and second round of amplification ( 1° PCR: mIghd-114-rv, 5'- SEQ ID NO: 145 -3'; 2° PCR: mIghd-079-rv, 5'-SEQ ID NO: 146 -3'). Sequencing was performed on 454 GS FLX+ and Illumina MiSeq 2x300, data processing was done using sciReptor, versions v1.0.2-3-ga48fc90 and v1.1-2-gf4cf8e2, respectively.

### Example 1.6. Ig Gene Cloning and Recombinant Antibodies Production.

Ig gene cloning and recombinant antibody production were performed as described before. In brief, templates from the first scPCR round were amplified with primers containing linker sequences and restrictions sites for cloning. The amplicons were cloned into human IGHG1 (ENA: LT615368; Addgene ID: 80795) and IGKC (ENA: LT615369; Addgene ID: 80796) expression vectors, respectively, and subsequently Sanger sequenced. The corresponding heavy and light chain plasmids were co-transfected into HEK293T cells. Recombinant antibodies were semipurified from supernatants using Amicon MWCO 30kDa Ultra Centrifugal Filters (Millipore). IgG concentrations were determined by ELISA.

For functional *in vivo* experiments, the V regions of the respective Ig heavy and Ig light chain plasmids encoding the A12 and B1.8 antibodies were subcloned into mouse *Ighg2b* (ENA: LR588430; Addgene ID: 127155), Igkc (ENA: LR588433; Addgene ID: *127157*) *and Iglc2* (ENA: LR588434; Addgene ID: 127156) expression vectors, respectively. Expression and purification of recombinant murine IgG2b antibodies was done as described above.

### Example 1.7. Antibodies Reactivity Testing by ELISA.

96 well plates (Costar) were coated overnight at 4 °C with MDA-LDL (3 µg/ml, Holzel), native LDL (3 µg/ml, Holzel), Hsp60 (1 µg/ml, Enzo) or ALDH4A1-FLAG (5 µg/ml) and were subsequently blocked for 1 h with 1% BSA (Sigma). Coated plates were incubated with the recombinant monoclonal antibodies at the indicated concentrations for 1.5 hours at RT. Bound antibodies were detected by goat anti-human IgG secondary antibody coupled to horse-radish-peroxidase (HRP) (Jackson Immuno Research) diluted 1/1,000 in 1% BSA in PBS which was then detected using an ABTS substrate solution (Roche). The OD at 405 nm was determined on a Benchmark Plus microplate spectrophotometer (Bio-Rad). mGO53 was used as a negative control.

### Example 1.8. Polyreactivity ELISA.

Polyreactivity ELISAs with positive (ED38 and JB40) and negative (mGO53) control monoclonal antibodies were performed without BSA block after coating with insulin (10 µg/ml, Fitzgerald), double-stranded DNA (10 µg/ml, Sigma) or LPS 10 µg/ml, Sigma L2630) as previously described.

### Competition ELISA

For competition immunoassays, A12 antibody (1 µg/ml) was preincubated with increasing concentrations (0, 50, 100, 200, 400 µg/ml) of the indicated competitors (BSA, MDA-LDL or ALDH4A1-FLAG) overnight at 4 °C. A12-competitor solutions were added to ALDH4A1-FLAG protein (5 µg/ml) coated ELISA plates, and bound A12 antibody was detected as described above. Results are expressed as ratios of A12 binding to ALDH4A1-FLAG in the presence (B) or absence (B0) of the competitor.

### Example 1.9. Immunofluorescence.

For tissue immunofluorescence, spleen, heart, aorta and liver specimens were fixed with 4% paraformaldehyde, incubated with 30% sucrose, embedded in OCT compound (Olympus), and frozen in dry ice. 10 µM sections were permeabilized with 0.5% Triton X-100 (Sigma) and blocked with 2% BSA (Sigma) and 5% Normal Serum (Sigma). The following commercial primary anti-mouse antibodies were used: Rat anti-B220 (BD, 1/100), rabbit anti-oxLDL (Acris, 1/400), mouse anti-SMA-1 biotin (ThermoFisher Scientific, 1/200) and rat anti-MAC-2 (TebaBio, 1/200); and the following fluorophore-conjugated secondary antibodies (Molecular Probes, 1/500) or streptavidins: goat anti-rat Alexa Fluor 488 or 568, goat anti-rabbit Alexa Fluor 568 and streptavidin Alexa Fluor 488. Germinal center B cells were stained using lectin PNA Alexa Fluor 647 (Life Technologies). Recombinantly produced antibodies (human IgG1, 8-10 µg/ml) were used in combination with goat anti-human IgG Alexa Fluor 488 or 647 secondary antibodies (Jackson ImmunoResearch, 1/500). Slides were mounted with Prolong Gold (Life Technologies) after Dapi staining and images were taken using a Leica SPE confocal microscope.

### Example 1.10. Immunoprecipitation with Recombinant Antibodies and Proteomics Analysis.

Aortas from LDLR-/- HFD (n=27) and LDLR+/+ ND (n=30) mice were homogenized in 1% NP-40, 20 mM Tris-HCl pH 8, 150 mM NaCl, 5% glycerol lysis buffer supplemented with protease and phosphatase inhibitors cocktail (Roche) and the amount of protein was quantified using the Pierce BCA Protein Assay Kit (Thermo Fisher Scientific). In parallel, recombinant monoclonal antibodies (A10, A11, A12, D7CT, E9CT and B1.8) were covalently coupled onto an amine-reactive resin using the Pierce Co-Immunoprecipitation Kit (Thermo Fisher Scientific), in accordance with the manufacturer's instructions. 2 mg of protein extracts were pre-cleared by incubating with 50 µl of underivatized agarose beads (Co-IP, Pierce) for 30 min at 4°C. The supernatant was further incubated with 20 µg of resin-coupled antibody overnight at 4 °C. Beads were washed four times with lysis buffer and two times with lysis buffer without detergent. Bound proteins were released from beads by boiling in sample buffer (5% SDS) for 5 min at 95 °C. Immunoprecipitates were resolved by SDS-PAGE and subjected to protein digestion followed by nanoliquid chromatography coupled to mass spectrometry for protein identification and quantification by spectral counting ³. Each sample was infused in triplicate. Peptide identification from MS/MS data was performed using the probability ratio method ⁴. False discovery rates (FDR) of peptide identifications were calculated using the refined method; 1%FDR was used as criterion for peptide identification.

### Example 1.11. Validation of proteomics results by Immunoprecipitation and Immunobloting.

Aortas from LDLR-/- HFD (n=8) were homogenized in 1% NP-40 lysis buffer supplemented with protease and phosphatase inhibitors cocktail (Roche) as in the proteomics experiment. In parallel, recombinant antibodies A12 and B1.8, and an anti-ALDH4A1 antibody (Abcam) were covalently coupled onto an amine-reactive resin using the Pierce Co-Immunoprecipitation Kit (Thermo Fisher Scientific). 1 mg of total protein from aorta lysates were used to immunoprecipitate with 10 µg of resin-coupled antibody overnight at 4 °C. Elution was done with pH 2.8 elution buffer. Immunoprecipitates were size-fractionated on SDS-PAGE 12% acrylamide-bisacrylamide gels and transferred to Protan nitrocellulose membrane (GE Healthcare). Membrane was probed with rabbit anti-ALDH4A1 (1/10,000, Abcam). Then, the membrane was incubated with anti-rabbit IgG HRP TrueBlot (1/1000, Rockland) and developed with Amersham ECL Western Blotting Detection Reagent (GE Healthcare Life Sciences).

Moreover, HEK293T cells transfected with murine ALDH4A1-FLAG, as detailed below, were lysed in 1% NP-40 lysis buffer supplemented with protease and phosphatase inhibitors cocktail (Roche), and were used to performed IP with additional recombinant antibodies (A4, A5, A12, B8, B10, C5, B1.8) and with anti-ALDH4A1 antibody (Abcam) covalently coupled onto an amine-reactive resin (Pierce Co-Immunoprecipitation Kit; Thermo Fisher Scientific). 200 µg of total protein were used for each IP.

### Example 1.12. Quantitative Proteomics Analysis of Human Aortas.

Tissue (100 mg of FL, FS or H) was homogenized at low temperature, and lysates were resuspended in buffer (50 mM iodoacetamide (Sigma), 1% SDS, 1mM EDTA, 100 mM Tris-HCL, pH 8.5) for protein extraction. Proteins were extracted by vortexing samples 4 times with 15min intervals on ice. Proteins in the supernatant were measured by the BCA method and stored at - 80°C until proteomics analysis.

Tissue protein samples were subjected to filter-aided digestion with trypsin according to manufacter's instructions (Nanosep Centrifugal Devices with Omega Membrane-10K, PALL), and the resulting peptides to multiplexed isobaric labeling with TMT reagents (Thermo Fisher Scintific). Two of the 10 channels were reserved for internal reference standard samples created by pooling the samples. Tissue peptides were separated into eight fractions using OASIS MCX cartridges. Tissue fractions were analysed on an Easy nLC 1000 liquid chromatography system coupled to an Orbitrap Fusion mass spectrometer.

Quantitative proteomics by multiplexed isobaric labelling and quantitative analysis of the data are performed following detailed protocols set up in Jesus Vazquez lab and already described. Identification, quantification and statistical and systems biology analysis are done using models developed in Jesus Vazquez lab and fully described before. Mass spectrometry analysis was performed in a hybrid quadrupole-orbitrap machine (HF Orbitrap, ThermoFisher).

### Example 1.13. ALDH4A1 recombinant protein production and purification.

Mouse ALDH4A1 ORF mammalian expression plasmid, C-Flag tag (Sino Biological) was transfected in HEK293T cells using Polyethylenimine (PEI, Sigma). After 72 h, cells were lysed with 50 mM Tris HCl pH 7.4, 150 mM NaCl, 1 mM EDTA and 1% TRITON X-100 lysis buffer supplemented with protease and phosphatase inhibitors cocktail (Roche). ALDH4A1-FLAG protein was then purified using the ANTI-FLAG M2 Affinity Gel (Sigma), in accordance with the manufacturer's instructions. Elution was performed with the 3X FLAG peptide (Sigma), which was then removed using 30 kDa Microcon Centrifugal Filters (Millipore).

### Example 1.14. Quantification of ALDH4A1 protein levels in plasma by ELISA.

Plasma from LDLR-/- HFD (n=11) and LDLR+/+ ND mice (n=10) were used to quantify murine ALDH4A1 protein levels using the Mouse P5CDH/ALDH4A1 ELISA Kit (LifeSpan BioSciences), in accordance with the manufacturer's instructions. Plasmas were used at 1/4 dilution. In addition, plasma from human carotid atherosclerosis patients (n=63) and controls (n=54) were used to quantify human ALDH4A1 protein levels using the Human PSCDH/ALDH4A1 ELISA Kit (LifeSpan BioSciences), at 1/10 dilution.

### Example 1.15. Immunohistochemistry.

Mouse hearts and livers were fixed in neutral-buffered 10% formalin solution (Sigma), embedded in paraffin blocks, and cut in 10 µM sections. For immunohistochemistry, sodium citrate buffer was used for antigen retrieval and rabbit anti-ALDH4A1 (1/100, Abcam) antibody was used, followed by a biotinylated goat anti-rabbit antibody (1/200, Abcam). The biotinylated antibody was detected with the ABC system using diaminobenzidine as substrate (Vector Laboratories). Images were acquired with a Leica DM2500 microscope.

### Example 1.16. Immunohistochemistry in humans.

Human atherosclerotic plaques were fixed in 3.7 % paraformaldehyde, embedded in paraffin and sectioned at 3µm. Sections were deparaffinised, hydrated and incubated with 6% goat serum, BSA 4% in PBS for 1h. The following primary antibodies were used: A12 and B1.8 (10 µg/ml, overnight). In addition, an irrelevant immunoglobulin (Dako) was used at the same concentration in order to assess non-specific staining. Sections were then incubated with antimouse biotinylated secondary antibody (1:250 dilution, 1h, Dako) and ABComplex (30 min, Vector lab), followed by staining with 3,30-diaminobenzidine (DAB), hematoxylin counterstaining, and mounting in DPX medium.

### Example 1.17. Atherosclerosis development quantification.

LDLR-/- mice were injected intravenously with mA12-IgG2b (n=16), mB1.8-IgG2b (n=16) or PBS (n=15), 100 µg antibody every 10 days over 12 weeks, in parallel to HFD treatment. Saline-perfused hearts were fixed with 4% paraformaldehyde, incubated with 30% sucrose, embedded in OCT compound (Olympus), and frozen in dry ice. Atherosclerotic plaque area was quantified by ImageJ analysis of Oil-Red-O-stained serial 80 µm-spaced cryostat 10 µm cross sections of aortic sinus, starting from proximal aorta to the heart apex ¹⁵. The collagen content within atherosclerotic lesions was quantified from Picrosirius Red-stained cross sections. Plasma levels of Free Cholesterol and LDL were measured using Dimension RxL Max Analyzer (Siemens).

### Evaluation of A12 antibody at the atheroma plaque of infused mice

10-week-old LDLR-/- male mice were fed with HFD for 12 weeks, infused intravenously with 100 µg of huA12-IgG1 (n=5), huB1-8-IgG1 (n=4) or PBS (n=4), and sacrificed 3-7 days later. Saline-perfused hearts were fixed with 4% paraformaldehyde, incubated with 30% sucrose, embedded in OCT compound (Olympus), and frozen in dry ice. 10 µm cross sections of aortic sinus were stained with goat anti-human IgG Alexa Fluor 647 antibody (Jackson ImmunoResearch, 1/500) to detect the injected antibody by immunofluorescence.

### Example 1.18. Statistical analysis.

Statistical analysis was performed in GraphPad Prism 7.3. In most cases, the graphs show the mean and, when shown, the errors bars represent standard deviation (SD). For values that came from a Gaussian distribution (D'Agostino-Pearson omnibus normality test), the two-tailed Student t-test was used (P value ≤0.05 was considered significant) when comparing two groups. One-way or two-way ANOVA test (q≤0.05 was consider significant, after correcting for multiple comparison using the original FDR method of Benjamini and Hochberg) was used when comparing more than two groups or different time points, respectively. When the values did not come from a Gaussian distribution, the two-tailed Mann-Whitney test was used (P value ≤ 0.05 was consider significant), when comparing two groups. For continuous data, the Fisher Exact test for categorical data was used (P value ≤ 0.05 was consider significant). In human atherosclerosis patients, logistic regression analysis adjusted with cardiovascular risk factors was performed with atherosclerosis presence as dependent variable.

## Claims

1. Antibody or antibody fragment binding ALDH4A1 **characterized in that** it comprises a light chain variable region (VL) and a heavy chain variable region (VH), wherein said VH comprises HCDR1, HCDR2 and HCDR3 polypeptides and VL comprises LCDR1, LCDR2 and LCDR3 polypeptides and, wherein HCDR1 comprises the sequence SEQ ID NO: 1, HCDR2 comprises the sequence SEQ ID NO: 2, HCDR3 comprises the sequence SEQ ID NO: 3, LCDR1 comprises the sequence SEQ ID NO: 4, LCDR2 comprises the sequence SEQ ID NO: 5 and LCDR3 comprises the sequence SEQ ID NO: 6, for use in a method for *in vivo* imaging of atherosclerosis lesions in a patient.

2. Antibody for use, according to claim 1, in a method for *in vivo* diagnosis of atherosclerosis.

3. Antibody for use, according to any of the claims 1 or 2, wherein the method comprises the visualization of the atherosclerosis lesions by detection of the antibody previously administered to the patient.

4. Antibody for use, according to any of the claims 1 to 3, wherein the method comprises the following steps: a) Intravenous injection of the antibody for *in vivo* imaging into the patient, b) Visualization of the atherosclerosis lesions by detection of the antibody of step a) in the patient.

## Patentansprüche

1. Antikörper oder Antikörperfragment, der/das ALDH4A1 bindet, **dadurch gekennzeichnet, dass** er/es eine variable Region der leichten Kette (VL) und eine variable Region der schweren Kette (VH) umfasst, wobei die VH HCDR1-, HCDR2- und HCDR3-Polypeptide umfasst und die VL LCDR1-, LCDR2- und LCDR3-Polypeptide umfasst und wobei HCDR1 die Sequenz SEQ ID NO: 1 umfasst, HCDR2 die Sequenz SEQ ID NO: 2 umfasst, HCDR3 die Sequenz SEQ ID NO: 3 umfasst, LCDR1 die Sequenz SEQ ID NO: 4 umfasst, LCDR2 die Sequenz SEQ ID NO: 5 umfasst und LCDR3 die Sequenz SEQ ID NO: 6 umfasst, zur Verwendung in einem Verfahren zur In-vivo-Bildgebung von Atheroskleroseläsionen bei einem Patienten.

2. Antikörper zur Verwendung nach Anspruch 1 in einem Verfahren zur *In-*vivo-Diagnose von Atherosklerose.

3. Antikörper zur Verwendung nach einem der Ansprüche 1 oder 2, wobei das Verfahren die Visualisierung der Atheroskleroseläsionen durch Nachweis des dem Patienten zuvor verabreichten Antikörpers umfasst.

4. Antikörper zur Verwendung nach einem der Ansprüche 1 bis 3, wobei das Verfahren die folgenden Schritte umfasst: a) Intravenöse Injektion des Antikörpers in den Patienten zur In-vivo-Bildgebung, b) Visualisierung der Atheroskleroseläsionen durch Nachweis des Antikörpers aus Schritt a) im Patienten.

## Revendications

1. Anticorps ou fragment d'anticorps se liant à l'ALDH4A1 **caractérisé en ce qu'**il comprend une région variable de chaîne légère (VL) et une région variable de chaîne lourde (VH), dans lequel ladite VH comprend les polypeptides HCDR1, HCDR2 et HCDR3 et la VL comprend les polypeptides LCDR1, LCDR2 et LCDR3 et, dans lequel HCDR1 comprend la séquence SEQ ID NO : 1, HCDR2 comprend la séquence SEQ ID NO :n 2, HCDR3 comprend la séquence SEQ ID NO : 3, LCDR1 comprend la séquence SEQ ID NO : 4, LCDR2 comprend la séquence SEQ ID NO : 5 et LCDR3 comprend la séquence SEQ ID NO : 6, destiné à être utilisé dans une méthode d'imagerie *in vivo* des lésions d'athérosclérose chez un patient.

2. Anticorps destiné à être utilisé, selon la revendication 1, dans un procédé de diagnostic in vivo de l'athérosclérose.

3. Anticorps à utiliser, selon l'une quelconque des revendications 1 ou 2, dans lequel le procédé comprend la visualisation des lésions d'athérosclérose par la détection de l'anticorps précédemment administré au patient.

4. Anticorps destiné à être utilisé, selon l'une des revendications 1 à 3, dans lequel le procédé comprend les étapes suivantes : a) Injection intraveineuse de l'anticorps pour l'imagerie *in vivo* dans le patient, b) Visualisation des lésions d'athérosclérose par détection de l'anticorps de l'étape a) chez le patient.
